# EUROPÄISCHE PATENTSCHRIFT

(11) **EP 1 897 568 B1**
(45) Veröffentlichungstag und Bekanntmachung des Hinweises auf die Patenterteilung: **23.06.2010**
(21) Anmeldenummer: 07014369.8
(22) Anmeldetag: 21.07.2007
(51) Int. Cl.: A61M 1/00

(54) **Chirurgisches System**
Surgical system
Système chirurgical

(30) Priorität: 08.09.2006 DE 102006042815; 17.11.2006 DE 102006054628
(43) Veröffentlichungstag der Anmeldung: 12.03.2008
(73) Patentinhaber: Carl Zeiss Surgical GmbH, 73447 Oberkochen (DE)
(72) Erfinder: Kübler, Christoph, 73447 Oberkochen (DE); Eichler, Michael J., 73457 Essingen (DE); Maier, Tobias, 73430 Aalen (DE)
(74) Vertreter: Gauss, Nikolai

(56) Entgegenhaltungen:
- US-A- 4 496 342
- US-A- 4 832 685
- US-A1- 2002 004 657

## Beschreibung

Die Erfindung betrifft ein chirurgisches System zur Steuerung von Fluid bei der Behandlung eines Kataraktes mit der Phakoemulsifikationstechnik.

Zur Behandlung einer Linsentrübung, welche in der Medizin als Grauer Star bezeichnet wird, gibt es mehrere chirurgische Techniken. Die am weitesten verbreitete Technik ist die Phakoemulsifikation, bei der eine dünne Spitze in die erkrankte Linse eingeführt und mit Ultraschall zu Schwingungen angeregt wird. Die vibrierende Spitze emulsifiziert in ihrer nächsten Umgebung die Linse derart, dass die entstehenden Linsenfragmente durch eine Leitung von einer Pumpe abgesaugt werden können. Ist die Linse vollständig emulsifiziert worden, kann in den leeren Kapselsack eine neue künstliche Linse eingesetzt werden, so dass ein derart behandelter Patient wieder ein gutes Sehvermögen erreichen kann.

Bei der Phakoemulsifikation kommt eine Vorrichtung zum Einsatz, welche allgemein eine schwingfähige Spitze in einem Handstück, eine Spülleitung (Irrigationsleitung) für die Zufuhr von Spülfluid zu der zu behandelnden Linse und eine Saugleitung (Aspirationsleitung) zum Abtransportieren emulsifizierter Linsenfragmente in einen Sammelbehälter aufweist. Während des Abtransportierens in den Sammelbehälter kann es vorkommen, dass ein Linsenfragment den Eingangsbereich der Handstückspitze verstopft. Bei kontinuierlich laufender Saugpumpe baut sich somit stromabwärts in der Aspirationsleitung ein Vakuum auf. Durch zum Beispiel fortgesetzte Ultraschallschwingungen der Spitze kann das Linsenfragment in kleinere Segmente zerbrechen, wodurch die Verstopfung (Okklusion) schlagartig beendet ist. Der aufgebaute Unterdruck in der Aspirationsleitung führt dazu, dass bei einem solchen Okklusionsdurchbruch in sehr kurzer Zeit eine relativ große Fluidmenge aus dem Auge gesaugt wird. Dies kann zur Folge haben, dass ein Kollaps der Augenvorderkammer eintritt. Es ist dabei möglich, dass der Kapselsack zur Handstückspitze gezogen und von der Spitze durchstochen wird. Neben einer solchen Verletzung des Kapselsackes kann ferner eine zu tief eingedrungene Spitze eine Beschädigung des hinter dem Kapselsack liegenden Augen-Glaskörpers bewirken.

Im Stand der Technik werden verschiedene Lösungen vorgeschlagen, um bei einem Okklusionsdurchbruch einen Kollaps der Augenvorderkammer zu vermeiden. In US 4,832,685 lässt sich die Aspirationsleitung mit der Irrigationsleitung verbinden, so dass ein Druckausgleich durch das Irrigationsfluid erreicht wird. Nachteilig ist dabei, dass das in der Irrigationsleitung vorhandene Fluid zu Druckschwankungen angeregt wird. Dies führt zu einer zusätzlichen Destabilisierung des Druckes in der Augenvorderkammer. Ein weiterer Nachteil besteht darin, dass bei einem derartigen Fluid-Druckausgleich kontaminiertes Fluid aus der Aspirationsleitung in die Irrigationsleitung fließen kann. Ein solches chirurgisches System lässt sich daher nur für einen einzelnen Patienten verwenden.

Eine andere Möglichkeit besteht darin, einen Druckausgleich mittels Umgebungsluft durchzuführen. In die Aspirationsleitung wird dabei Luft mit atmosphärischem Druck eingeleitet. Vorteilhaft ist dabei, dass es zu keiner Anregung einer Druckschwankung in der Irrigationsleitung kommt. Die in die Aspirationsleitung eingebrachte Luft verändert jedoch die fluidischen Eigenschaften des Ansaugsystems, so dass die Luft aus der Aspirationsleitung gepumpt werden muss, um wieder eine dynamische Saugdruckkennlinie in der Aspirationsleitung zu erzielen.

In US 6,740,074 B2 und US 6,261,283 wird vorgeschlagen, aus einem am Ende der Aspirationsleitung angeordneten Sammelbehälter Fluid zu entnehmen und in die Aspirationsleitung zu führen. Bei dieser Lösung werden jedoch kontaminierte Partikel aus dem Sammelbehälter in die Aspirationsleitung gebracht, so dass ein solches System unsteril wird und nicht für mehrere Patienten, sondern nur für einen einzelnen Patienten geeignet ist.

Es ist daher eine Aufgabe der Erfindung, ein chirurgisches System zu schaffen, welches bei einem Unterdruck in einer Aspirationsleitung einen schnellen Druckausgleich ermöglicht, wobei keine Druckschwankungen in der Irrigationsleitung induziert werden, dabei die fluidischen Eigenschaften in der Aspirationsleitung nicht verändert werden, und dabei kein kontaminiertes Fluid in die Irrigationsleitung gelangt, so dass das System auch für mehrere Patienten verwendet werden kann.

Die Aufgabe wird durch ein System mit den Merkmalen des unabhängigen Anspruchs 1 gelöst. Vorteilhafte Weiterbildungen der Erfindung sind in den Unteransprüchen beschrieben.

Das erfindungsgemäße chirurgische System zur Steuerung eines Fluids weist auf: Eine Irrigationsleitung, welche an einem Ende mit einem ersten Fluidbehälter zur Aufnahme von Irrigationsfluid und an einem anderen Ende mit einem chirurgischen Handstück verbunden ist, eine Saugpumpe, eine Aspirationseingangsleitung, welche vom chirurgischen Handstück zu einem Eingang der Saugpumpe so vorgesehen ist, dass sich von der Saugpumpe Fluid durch das Handstück saugen lässt, einen Drucksensor, welcher den Fluiddruck in der Aspirationseingangsleitung erfasst, eine Aspirationsausgangsleitung, welche einen Ausgang der Saugpumpe mit einem Sammelbehälter so verbindet, dass Fluid von dem Ausgang der Saugpumpe in den Sammelbehälter zuführbar ist, einen zweiten Fluidbehälter zur Aufnahme von Irrigationsfluid, eine Aspirationsbelüftungsleitung, welche den zweiten Fluidbehälter mit der Aspirationseingangsleitung verbindet, und ein Belüftungsventil, welches in der Aspirationsbelüftungsleitung vorgesehen ist und sich in Abhängigkeit vom Fluiddruck in der Aspirationseingangsleitung schalten lässt.

Bei einer Okklusion in der Aspirationsleitung kann mit dem erfindungsgemäßen System Fluid aus dem zweiten Fluidbehälter durch die Aspirationsbelüftungsleitung in die Aspirationseingangsleitung geführt werden. Steigt zum Beispiel nach einem Okklusionsdurchbruch der Vakuumdruck in der Aspirationsleitung wieder in Richtung zum normalen Saugdruck an, lässt sich durch das Belüftungsventil die Belüftungsleitung entsprechend freischalten, so dass ein rascher Druckausgleich möglich ist und ein Abfallen des Saugdruckes auf einen zu hohen Wert vermieden wird. Das zugeführte Fluid stammt nicht aus dem ersten Fluidbehälter, der das Irrigationsfluid enthält und mit der Irrigationsleitung verbunden ist. Durch den zweiten Fluidbehälter wird eine vollständige Trennung von diesem ersten Fluidbehälter erreicht, so dass während des Belüftens keine Druckschwankungen in der Irrigationsleitung angeregt werden können. Ferner ist durch die Trennung der beiden Fluidbehälter eine Kontamination der Irrigationsleitung ausgeschlossen. Da der zweite Fluidbehälter steriles Fluid enthält, ist auch eine Kontamination der Aspirationsleitung durch das Belüften ebenfalls ausgeschlossen. Somit ist es möglich, das chirurgische System auch ohne Gefahr einer Kontamination mit zuvor eingebrachten Verunreinigungen bei mehreren Patienten zu verwenden, die aufeinander folgend behandelt werden.

Gemäß der Erfindung ist der zweite Fluidbehälter durch eine Befüllungsleitung, welche ein Befüllungsventil aufweist, befüllbar, wobei die Befüllungsleitung an einem Ende mit der Irrigationsleitung verbunden ist. Somit genügt es, nur den ersten Fluidbehälter mit Irrigationsfluid zu füllen, so dass anschließend aus diesem sterilen Fluid der zweite Fluidbehälter befüllt werden kann. Ein solches Befüllen des zweiten Fluidbehälters kann zum Beispiel vor Beginn einer Operation erfolgen. Durch das Befüllungsventil wird eine zuverlässige Trennung zwischen der Irrigationsleitung und dem Teil der Befüllungsleitung erreicht, der zum zweiten Fluidbehälter zugewandt ist. Vorzugsweise endet das andere Ende der Befüllungsleitung in einem vorbestimmten Abstand zu einer maximalen Fluidpegelhöhe des zweiten Fluidbehälters, so dass kein direkter Berührkontakt zwischen dem anderen Ende der Befüllungsleitung und dem Fluid im zweiten Fluidbehälter gegeben ist. Damit kann eine Kontamination der Irrigationsleitung noch sicherer ausgeschlossen werden.

Ist ein Sensor zum Detektieren eines Fluidpegels im zweiten Fluidbehälter vorgesehen, kann eine Abnahme des Fluidpegels nach einem Belüften der Aspirationseingangsleitung erfasst und zu einem geeigneten Zeitpunkt ein Nachfüllen des zweiten Fluidbehälters veranlasst werden. Um die Sicherheit beim Nachfüllen zu erhöhen, kann der zweite Fluidbehälter mit einer Überlaufleitung verbunden sein, durch welche überschüssiges Fluid in einen zusätzlichen Behälter ableitbar ist.

Werden die Irrigations- und Aspirationsleitungen sowie der Fluidbehälter nur für jeweils einen Patienten verwendet, ist die Gefahr einer Kontamination des zweiten Fluidbehälters geringer, so dass bei einer anderen Ausführungsform vorgesehen sein kann, die Befüllungsleitung an dem anderen Ende mit dem zweiten Fluidbehälter zu verbinden. Eine solche Verbindung zwischen dem anderen Ende und dem zweiten Fluidbehälter ist durch mechanische oder chemische Verbindungsmittel oder durch ein thermisches Verbindungsverfahren erzielbar. Es kann sich zum Beispiel um Klemmen, Kleben, Schweißen und besonders bei Kunststoffen durch ein Verbinden mittels Polymerisation oder Polykondensation handeln.

Ist die Befüllungsleitung mit dem zweiten Fluidbehälter verbunden, lässt sich ein geschlossenes System bilden. Wird Fluid aus dem zweiten Fluidbehälter abgelassen, muss innerhalb des zweiten Fluidbehälters ein Druckausgleich erfolgen. Gemäß einer bevorzugten Ausführungsform lässt sich dies dadurch erreichen, dass der zweite Fluidbehälter eine Wandung mit mindestens einem elastisch verformbaren Bereich aufweist. Die Verringerung der Fluidmenge innerhalb des zweiten Fluidbehälters führt dann zu einer Verformung des elastischen Bereiches, so dass keine Druckluft oder ähnliches in den zweiten Fluidbehälter nachgeführt werden muss. Damit lässt sich das System relativ einfach betreiben.

Vorzugsweise ist der zweite Fluidbehälter rohrförmig zum Beispiel als Schlauch wie die Befüllungsleitung oder Aspirationsbelüftungsleitung ausgebildet, wodurch eine kostengünstige und einfach zu realisierende Lösung erreicht wird. Der Schlauch bietet ferner den Vorteil, dass er nicht nur in einem begrenzten Bereich, sondern entlang seines gesamten Umfangs elastisch verformbar ist. Besonders bevorzugt sind die Befüllungsleitung, der zweite Fluidbehälter und die Aspirationsbelüftungsleitung einstückig ausgebildet, so dass eine einfach Montage in einer Kassette erreicht wird.

Weist die Irrigationsleitung ein Irrigationsventil auf, kann dieses in eine solche Stellung gebracht werden, dass die Irrigationsleitung unterbrochen ist. Ein Befüllen des zweiten Fluidbehälters mit Fluid aus dem ersten Fluidbehälter lässt sich dann mittels der Befüllungsleitung besonders geschickt durchführen, wenn das Irrigationsventil in der Irrigationsleitung zwischen Handstück und Befüllungsleitung angeordnet ist. In diesem Fall treten beim Befüllen des zweiten Fluidbehälters keine Druckschwankungen in dem Teil der Irrigationsleitung auf, der zwischen Irrigationsventil und Handstück angeordnet ist. Somit ist sichergestellt, dass während des Befüllens des zweiten Fluidbehälters im Auge keine Druckschwankungen induziert werden.

Gemäß einer weiteren Ausführungsform der Erfindung ist Fluid von der Aspirationsbelüftungsleitung am Boden oder in der Nähe des Bodens des zweiten Fluidbehälters ableitbar. Dies stellt sicher, dass stets die maximale verfügbare Menge an Belüftungsfluid zur Verfügung gestellt werden kann und bei gefülltem Behälter zudem keine Luft in die Aspirationseingangsleitung zugeführt wird.

Vorzugsweise erfasst der Drucksensor den Fluiddruck in der Aspirationsleitung nahe am Handstück. Damit ist es möglich, dass bei einem Okklusionsdurchbruch aufgrund der kurzen Wegstrecke von Handstückspitze zu Drucksensor die Druckänderung schnell erfasst und somit eine Ansteuerung des Belüftungsventils schnell erfolgen kann. Besonders schnell ist eine Belüftung erreichbar, wenn der Drucksensor den Fluiddruck in der Aspirationsleitung innerhalb des Handstückes, vorzugsweise in der Nähe der Handstückspitze, erfasst.

Weitere Vorteile und Merkmale der Erfindung werden mit Bezug auf die nachfolgenden Zeichnungen erklärt, in welchen zeigen:
- Fig. 1: eine schematische Darstellung einer ersten Ausführungsform des chirurgischen Systems gemäß der Erfindung,
- Fig. 2: eine schematische Darstellung einer zweiten Ausführungsform des chirurgischen Systems gemäß der Erfindung,
- Fig. 3: schematische Darstellungen von mehreren Fluidbehältern in einem chirurgischen System gemäß der Erfindung, und
- Fig. 4: eine Darstellung des Druckverlaufes in der Aspirationsleitung des erfindungsgemäßen chirurgischen Systems in Abhängigkeit von der Zeit.

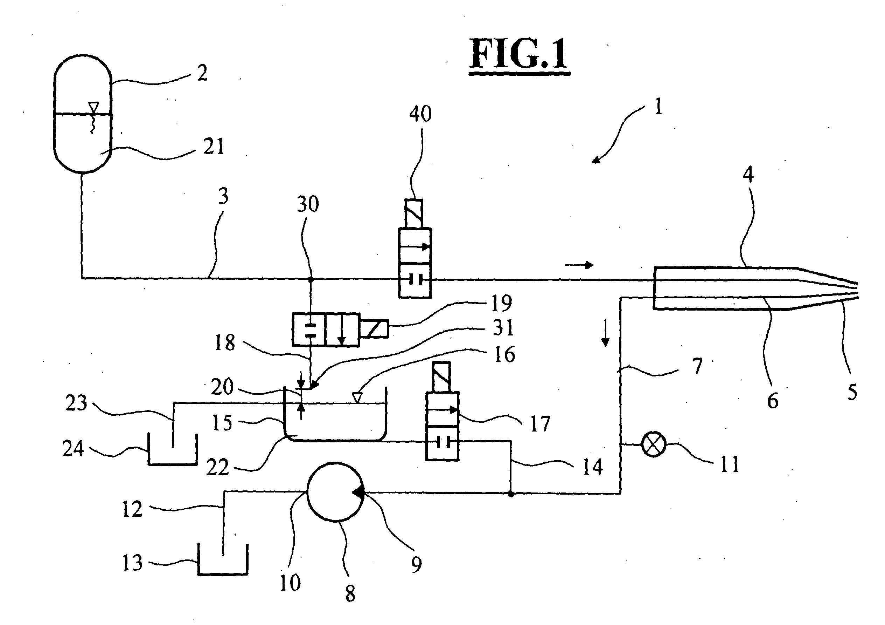

In Fig. 1 ist eine schematische Darstellung einer Ausführungsform des erfindungsgemäßen chirurgischen Systems 1 gezeigt. In einem ersten Fluidbehälter 2 ist ein Irrigationsfluid 21 enthalten, welches über eine Irrigationsleitung 3 zu einem chirurgischen Handstück 4 geleitet werden kann. Bei dem Handstück 4 kann es sich um ein Handstück zur Phakoemulsifikation handeln, bei dem eine vibrierende Spitze 5 eine getrübte Linse eines Auges emulsifiziert und die zertrümmerten Linsenfragmente abgesaugt werden. Ein Irrigationsventil 40, welches bei der Darstellung in Fig. 1 als 2-Wege-Ventil gezeigt ist, erlaubt einen Durchlass oder ein Sperren des Irrigationsfluides in Richtung zum Handstück 4. Von der Spitze 5 verläuft eine Aspirationsleitung 6 zu einem Ende des Handstückes 4, um emulsifizierte Linsenteile und Fluid aus dem Auge abzutransportieren. Der Abtransport wird durch eine Saugpumpe 8 verursacht, welche an ihrem Eingang 9 über eine Aspirationseingangsleitung 7 mit dem Handstück 4 verbunden ist. Ein Fluiddruck in der Aspirationseingangsleitung 7 wird durch einen Drucksensor 11 erfasst, der zwischen dem Eingang 9 der Saugpumpe 8 und dem Handstück 4 angeordnet ist. Vorzugsweise ist der Drucksensor 11 in der Nähe des Handstückes 4 vorgesehen, so dass eine Druckänderung im Bereich der Spitze 5 nach kurzer Wegstrecke durch das Handstück 4 detektiert werden kann. Eine noch schnellere Detektion einer Druckänderung wird erreicht, wenn der Drucksensor 11 den Fluiddruck in der Aspirationsleitung 6 innerhalb des Handstückes 4 erfasst. Die Aspirationsleitung 6 kann in diesem Fall als vorderer Abschnitt der Aspirationseingangsleitung 7 verstanden werden.

Die Saugpumpe 8 leitet die Linsengfragmente und Fluid an ihrem Ausgang durch eine Aspirationsausgangsleitung 12 in einen Sammelbehälter 13 weiter.

An die Aspirationseingansgleitung 7 ist eine Aspirationsbelüftungsleitung 14 angeschlossen, welche mit einem zweiten Fluidbehälter 15 verbunden ist. In dem zweiten Fluidbehälter 15 ist ein Fluid 22 enthalten, welches bei entsprechender Stellung eines in der Aspirationsbelüftungsleitung 14 vorgesehenen 2-Wege-Belüftungsventils 17 in die Aspirationseingangsleitung 7 zugeführt werden kann. Tritt innerhalb der Aspirationsleitungen 6 oder 7, zum Beispiel am distalen Ende der Aspirationsleitung im Bereich der Spitze 5 eine Verstopfung (Okklusion) durch zu große Linsenfragmente auf, so dass ein Absaugen durch die Aspirationsleitungen 6 und 7 blockiert ist, baut sich in diesen Leitungen ein Vakuumdruck auf. Dieser Druck kann mit dem Drucksensor 11 erfasst werden. Wenn die Okklusion durchbricht, steigt der Druck in der Leitung 7 vom Vakuumdruck wieder in Richtung zum Atmosphärendruck an. Bei dieser Druckänderung kann ein Kollaps der Augenvorderkammer auftreten. Um dies zu verhindern, wird unmittelbar nach Erfassen der Druckänderung von dem Drucksensor 11 das Belüftungsventil 17 so geschaltet, dass Fluid 22 vom zweiten Fluidbehälter 15 in die Aspirationsbelüftungsleitung 14 und dort in die Aspirationseingangsleitung 7 geleitet wird. Ein zu hohes Ansteigen des Druckes in der Aspirationseingangsleitung 7 wird damit verhindert.

Der zweite Fluidbehälter 15 ist mit einem Fluid 22 befüllt, welches bei der in Fig. 1 dargestellten Ausführungsform durch eine Befüllungsleitung 18 zugeführt wird. In der Befüllungsleitung 18 ist ein 2-Wege-Befüllungsventil 19 vorgesehen, welches den Fluiddurchgang sperrt oder freigibt. Die Befüllungsleitung 18 ist an einem Ende 30 mit der Irrigationsleitung 3 verbunden, so dass Fluid 21 in die Befüllungsleitung 18 zugeführt werden kann. Das andere Ende 31 der Befüllungsleitung 18 endet in einem vorbestimmten Abstand 20 zur maximalen Fluidpegelhöhe des zweiten Fluidbehälters 15, so dass kein direkter Berührkontakt zwischen dem anderen Ende 31 der Befüllungsleitung 18 und dem Fluid 22 im zweiten Fluidbehälter 15 gegeben ist. Somit liegt eine vollständige Trennung zwischen der Irrigationsleitung 3 und dem zweiten Fluidbehälter 15 beziehungsweise der Aspirationsbelüftungsleitung 14 und Aspirationseingansgleitung 7 vor. Eine Kontamination von unsterilem Fluid in der Aspirationseingangsleitung 7 in die Irrigationsleitung 3 ist somit auch bei durchgeschaltetem Befüllungsventil 19 ausgeschlossen.

Der zweite Fluidbehälter 15 ist mit einem Sensor 16 versehen, mit dem der Fluidpegel im zweiten Fluidbehälter 15 erfasst werden kann. Durch den Sensor 16 wird sichergestellt, dass die Befüllung des zweiten Fluidbehälters 15 nur bis zum Erreichen des maximal zulässigen Fluidpegels erfolgt. Zur weiteren Sicherheit besitzt der Fluidbehälter 15 noch einen Überlauf mit einer Überlaufleitung 23, durch welche überschüssiges Fluid in einen Behälter 24 geleitet werden kann. Die Behälter 24 und 13 können einstückig ausgebildet sein.

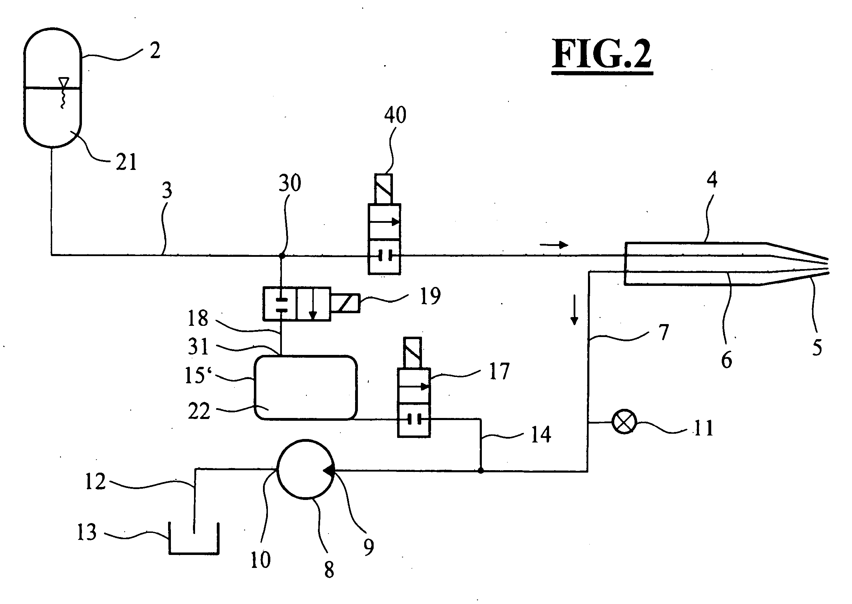

Bei der in Fig. 2 dargestellten zweiten Ausführungsform des chirurgischen Systems ist der zweite Fluidbehälter 15' ein geschlossener Behälter, wobei das andere Ende 31 der Befüllungsleitung 18 mit dem zweiten Fluidbehälter 15' verbunden ist. Um ein Entleeren des zweiten Fluidbehälters 15' zu ermöglichen, muss ein Druckausgleich innerhalb des Behälters vorgesehen werden. Dies lässt sich zum Beispiel dadurch erreichen, dass eine Wandung des zweiten Fluidbehälters 15' mit mindestens einem elastisch verformbaren Bereich versehen ist. Bei diesem elastisch verformbaren Bereich kann es sich um einen flexiblen Deckel auf einem starren Fluidbehälter 15' oder um eine flexible Seitenwand handeln. Ferner ist es möglich, dass der gesamte zweite Fluidbehälter 15' elastisch verformbar ausgebildet ist, zum Beispiel als Schlauch oder Beutel. Wird nach Schalten des Belüftungsventils 17 in eine geöffnete Position das Fluid 22 vom zweiten Fluidbehälter 15' in die Aspirationsbelüftungsleitung 14 transportiert, zieht sich der elastisch verformbare Bereich des zweiten Fluidbehälters 15' zusammen, wodurch das Fluidvolumen innerhalb des zweiten Fluidbehälters 15' verringert wird. Durch Schließen des Belüftungsventils 17 und nachfolgendes Öffnen des Befüllungsventils 19 kann der zweite Fluidbehälter 15' wieder vollständig mit Fluid 22 befüllt werden.

Die zweite Ausführungsform des in Fig. 2 dargestellten chirurgischen Systems unterscheidet sich von der in Fig. 1 dargestellten ersten Ausführungsform unter anderem dadurch, dass für den zweiten Fluidbehälter 15' ein Druckausgleich durch z. B. Atmosphärendruck nicht erforderlich ist. Außerdem kann auf den Sensor 16 für die Überwachung des Füllstandspegels, wie dies bei der in Fig. 1 gezeigten ersten Ausführungsform des chirurgischen Systems vorgesehen ist, verzichtet werden. Dies gilt auch für die Überlaufleitung 23 zum Ableiten von überschüssigem Fluid in den Behälter 24.

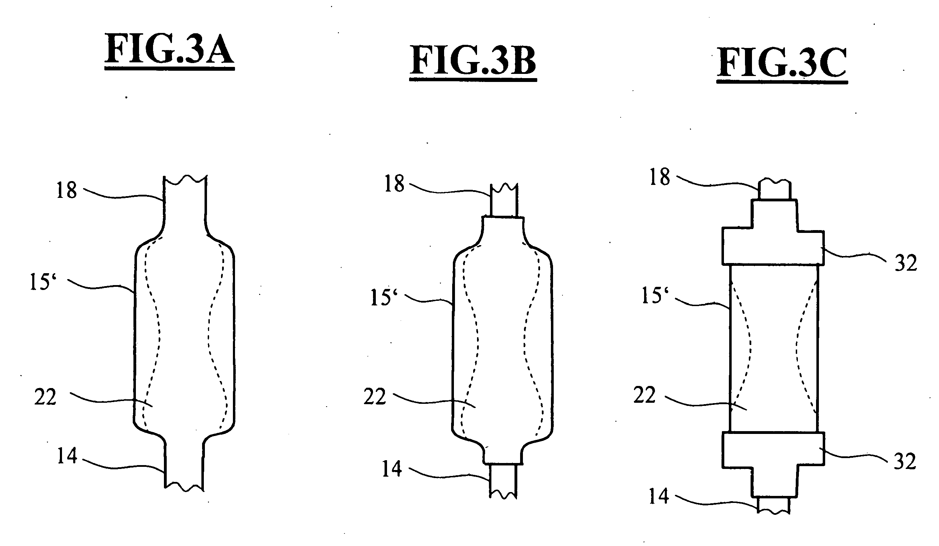

In Fig. 3 sind mehrere Formen eines Fluidbehälters 15' mit mindestens einem elastisch verformbaren Bereich dargestellt. Die Fluidbehälter 15' sind rohrförmig ausgebildet, wobei eine direkte Verbindung mit der Befüllungsleitung 18 und der Belüftungsleitung 14 besteht. Die Verbindung kann durch mechanische oder chemische Verbindungsmittel oder durch ein thermisches Verbindungsverfahren erzielt werden. Bei der in Fig. 3A dargestellten Form ist die Befüllungsleitung 18, der zweite Fluidbehälter 15' und die Belüftungsleitung 14 einstückig ausgebildet. Der Fluidbehälter 15' stellt somit einen im Durchmesser geweiteten Mittelabschnitt der Leitungen 18 und 14 dar. Bei der in Fig. 3B dargestellten Form handelt es sich bei dem zweiten Fluidbehälter 15' um ein Schlauchstück mit an den Enden verjüngten Durchmessern, welche mit den Leitungen 18 und 14 verbunden sind, während bei der in Fig. 3C dargestellten Form ein zusätzlicher Schlauch mit zugehörigen Reduzierstücken 32 vorgesehen ist. Die Fertigung der Fluidbehälter 15' kann zum Beispiel durch Extrusion, Blasformen oder ein Spritzgussverfahren erfolgen.

Wird Fluid 22 in die Belüftungsleitung 14 abgeführt, verringert sich bei einem geschlossenen Fluidbehälter 15' gemäß der zweiten Ausführungsform jeweils der Durchmesser im mittleren Bereich, siehe gestrichelte Linien in Fig. 3A bis 3C.

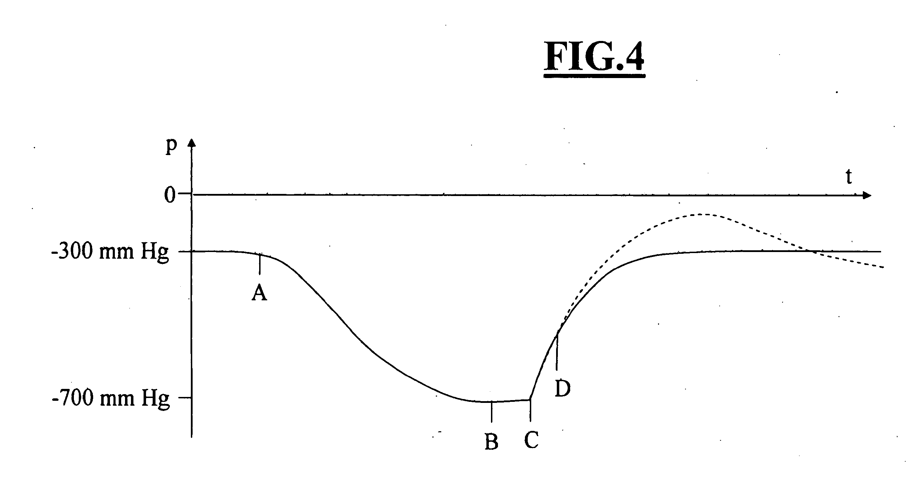

Beim Durchbrechen einer Okklusion wird vom Drucksensor 11 ein Ansteigen des Vakuumdruckes in Richtung zum Atmosphärendruck in der Aspirationseingangsleitung 7 erfasst. Daraufhin wird das Belüftungsventil 17 in eine Stellung bewegt, so dass die Aspirationsbelüftungsleitung freigeschaltet ist und Fluid 22 vom zweiten Fluidbehälter 15, 15' zur Aspirationseingangsleitung 7 geleitet werden kann. In Fig. 4 ist die Wirkung dieses Belüftungsvorganges mittels eines Graphen dargestellt. Das Diagramm zeigt einen Druckverlauf in einer Aspirationsleitung 6 oder 7 in Abhängigkeit von der Zeit. Vom üblichen Saugdruck in der Höhe von etwa -300 mm Hg nimmt nach Auftreten einer Okklusion, Ereignis "A", der Saugdruck in der Aspirationsleitung stetig zu, bis ein maximaler Vakuumdruck erreicht ist, Ereignis "B". Bricht die Okklusion auf, Ereignis "C", nimmt der Vakuumdruck in kurzer Zeit wieder in Richtung zum üblichen Saugdruck ab. Diese Druckänderung wird kurz darauf mittels des Drucksensors 11 erfasst, Ereignis "D", so dass das Irrigationsventil und das Belüftungsventil entsprechend geschaltet werden, um ein Befüllen der Aspirationseingangsleitung 7 zu erreichen. Anstatt einer Änderung des Vakuumdruckes auf Werte nahe dem Atmosphärendruck, siehe gestrichelter Linienverlauf in Fig. 4, wie dies beim Stand der Technik noch der Fall ist, wird durch das Befüllen der Aspirationseingangsleitung durch das erfindungsgemäße System ein Absinken des Vakuumdruckes nur bis zum üblichen Saugdruck von etwa -300 mm Hg erzielt. Das Befüllen lässt sich derart durchführen, dass der Druck nach weniger als 100 ms, bei geeigneter Platzierung des Drucksensors in der Nähe der Handstückspitze 5 bereits nach 30 ms, den üblichen Saugdruck von -300 mm Hg erreicht hat.

## Patentansprüche

1. Chirurgisches System (1) zur Steuerung eines Fluides, aufweisend:
- eine Irrigationsleitung (3), welche an einem Ende mit einem ersten Fluidbehälter (2) zur Aufnahme von Irrigationsfluid (21) und an einem anderen Ende mit einem chirurgischen Handstück (4) verbunden ist,
- eine Saugpumpe (8),
- eine Aspirationseingangsleitung (7), welche vom chirurgischen Handstück (4) zu einem Eingang (9) der Saugpumpe (8) so vorgesehen ist, dass sich von der Saugpumpe (8) Fluid durch das Handstück (4) saugen lässt,
- einen Drucksensor (11), welcher den Fluiddruck in der Aspirationseingangsleitung (7) erfasst,
- eine Aspirationsausgangsleitung (12), welche einen Ausgang (10) der Saugpumpe (8) mit einem Sammelbehälter (13) so verbindet, dass Fluid von dem Ausgang (10) der Saugpumpe (8) in den Sammelbehälter (13) zuführbar ist,
- einen zweiten Fluidbehälter (15; 15') zur Aufnahme von Irrigationsfluid (22),
- eine Aspirationsbelüftungsleitung (14), welche den zweiten Fluidbehälter (15; 15') mit der Aspirationseingangsleitung (7) verbindet,
- ein Belüftungsventil (17), welches in der Aspirationsbelüftungsleitung (14) vorgesehen ist und sich in Abhängigkeit vom Fluiddruck in der Aspirationseingangsleitung (7) schalten lässt,
**dadurch gekennzeichnet, dass** der zweite Fluidbehälter (15; 15') durch eine Befüllungsleitung (18), welche ein Befüllungsventil (19) aufweist, befüllbar ist, wobei die Befüllungsleitung (18) an einem Ende (30) mit der Irrigationsleitung (3) verbunden ist.

2. System (1) nach Anspruch 1, wobei ein anderes Ende (31) der Befüllungsleitung (18) in einem vorbestimmten Abstand (20) zu einer maximalen Fluidpegelhöhe des zweiten Fluidbehälters (15) endet, so dass kein direkter Berührkontakt zwischen dem anderen Ende (31) der Befüllungsleitung (18) und dem Fluid (22) im zweiten Fluidbehälter (15) gegeben ist.

3. System (1) nach einem der vorherigen Ansprüche, wobei ein Sensor (16) zum Detektieren eines Fluidpegels im zweiten Fluidbehälter (15) vorgesehen ist.

4. System nach einem der vorherigen Ansprüche, wobei der zweite Fluidbehälter (15) mit einer Überlaufleitung (23) verbunden ist, durch welche überschüssiges Fluid in einen Behälter (24) ableitbar ist.

5. System (1) nach Anspruch 1, wobei die Befüllungsleitung (18) an einem anderen Ende (31) mit dem zweiten Fluidbehälter (15') verbunden ist.

6. System (1) nach Anspruch 5, wobei eine Verbindung zwischen dem anderen Ende (31) der Befüllungsleitung (18) und dem zweiten Fluidbehälter (15') durch mechanische oder chemische Verbindungsmittel oder durch ein thermisches Verbindungsverfahren erzielbar ist.

7. System (1) nach einem der Ansprüche 5 oder 6, wobei der zweite Fluidbehälter (15') eine Wandung mit mindestens einem elastisch verformbaren Bereich aufweist.

8. System (1) nach einem der Ansprüche 5 bis 7, wobei der zweite Fluidbehälter (15') rohrförmig ausgebildet ist.

9. System (1) nach einem der Ansprüche 5 bis 8, wobei die Befüllungsleitung (18), der zweite Fluidbehälter (15') und die Aspirationsbelüftungsleitung (14) einstückig ausgebildet sind.

10. System (1) nach einem der vorherigen Ansprüche, wobei die Irrigationsleitung (3) ein Irrigationsventil (40) aufweist.

11. System (1) nach Anspruch 3, wobei das Irrigationsventil (40) in der Irrigationsleitung (3) zwischen Handstück (4) und Befüllungsleitung (18) angeordnet ist.

12. System (1) nach einem der vorherigen Ansprüche, wobei von der Aspirationsbelüftungsleitung (14) am Boden oder in der Nähe des Bodens des zweiten Fluidbehälters (15; 15') Fluid (22) ableitbar ist.

13. System (1) nach einem der vorherigen Ansprüche, wobei der Drucksensor (11) den Fluiddruck in der Aspirationsleitung (6, 7) nahe am Handstück (4) erfasst.

14. System (1) nach einem der vorherigen Ansprüche, wobei der Drucksensor (11) den Fluiddruck in der Aspirationsleitung (6, 7) innerhalb des Handstückes (4), vorzugsweise in unmittelbarer Nähe der Handstückspitze (5), erfasst.

## Claims

1. Surgical system (1) for controlling a fluid, comprising:
- an irrigation line (3), which is connected at one end to a first fluid container (2) for holding irrigation fluid (21), and which is connected at another end to a surgical handpiece (4),
- a suction pump (8),
- an aspiration inlet line (7), which is provided from the surgical handpiece (4) to an inlet (9) of the suction pump (8) such that fluid can be sucked through the handpiece (4) by the suction pump (8),
- a pressure sensor (11), which detects the fluid pressure in the aspiration inlet line (7),
- an aspiration outlet line (12), which connects an outlet (10) of the suction pump (8) to a collecting container (13) such that fluid can be delivered from the outlet (10) of the suction pump (8) into the collecting container (13),
- a second fluid container (15; 15') for holding irrigation fluid (22),
- an aspiration ventilation line (14), which connects the second fluid container (15; 15') to the aspiration inlet line (7),
- a venting valve (17), which is provided in the aspiration ventilation line (14) and can be switched depending on the fluid pressure in the aspiration inlet line (7),
**characterized in that** the second fluid container (15; 15') can be filled via a filling line (18), which has a filling valve (19), and the filling line (18) is connected at one end (30) to the irrigation line (3).

2. System (1) according to Claim 1, wherein another end (31) of the filling line (18) ends at a predefined distance (20) from a maximum fluid level height of the second fluid container (15), such that there is no direct physical contact between the other end (31) of the filling line (18) and the fluid (22) in the second fluid container (15).

3. System (1) according to one of the preceding claims, wherein a sensor (16) is provided for detecting a fluid level in the second fluid container (15).

4. System according to one of the preceding claims, wherein the second fluid container (15) is connected to an overflow line (23) through which excess fluid can be carried off into a container (24).

5. System (1) according to Claim 1, wherein the filling line (18) is connected at another end (31) to the second fluid container (15').

6. System (1) according to Claim 5, wherein a connection between the other end (31) of the filling line (18) and the second fluid container (15') can be achieved by mechanical or chemical connecting means or by a thermal connecting method.

7. System (1) according to one of Claims 5 and 6, wherein the second fluid container (15') has a wall with at least one elastically deformable area.

8. System (1) according to one of Claims 5 to 7, wherein the second fluid container (15') is tubular.

9. System (1) according to one of Claims 5 to 8, wherein the filling line (18), the second fluid container (15') and the aspiration ventilation line (14) are formed in one piece.

10. System (1) according to one of the preceding claims, wherein the irrigation line (3) has an irrigation valve (40).

11. System (1) according to Claim 3, wherein the irrigation valve (40) is arranged in the irrigation line (3) between handpiece (4) and filling line (18).

12. System (1) according to one of the preceding claims, wherein fluid (22) can be carried off by the aspiration ventilation line (14) at the bottom or near the bottom of the second fluid container (15; 15').

13. System (1) according to one of the preceding claims, wherein the pressure sensor (11) detects the fluid pressure in the aspiration line (6, 7) near the handpiece (4).

14. System (1) according to one of the preceding claims, wherein the pressure sensor (11) detects the fluid pressure in the aspiration line (6, 7) inside the handpiece (4), preferably in immediate proximity to the handpiece tip (5).

## Revendications

1. Système chirurgical (1) pour la commande d'un fluide, comprenant:
- une conduite d'irrigation (3), qui est raccordée par une extrémité à un premier réservoir de fluide (2) destiné à contenir un fluide d'irrigation (21) et par une autre extrémité à un instrument chirurgical (4),
- une pompe aspirante (8),
- une conduite d'entrée d'aspiration (7), qui est prévue de l'instrument chirurgical (4) à une entrée (9) de la pompe aspirante (8), de telle manière que du fluide puisse être aspiré par la pompe aspirante (8) à travers l'instrument (4),
- un capteur de pression (11), qui détecte la pression de fluide dans la conduite d'entrée d'aspiration (7),
- une conduite de sortie d'aspiration (12), qui relie une sortie (10) de la pompe aspirante (8) à un réservoir de collecte (13), de telle manière que du fluide puisse être envoyé de la sortie (10) de la pompe aspirante (8) dans le réservoir de collecte (13),
- un deuxième réservoir de fluide (15; 15') destiné à contenir un fluide d'irrigation (22),
- une conduite d'aération de l'aspiration (14), qui relie le deuxième réservoir de fluide (15; 15') à la conduite d'entrée d'aspiration (7),
- une soupape d'aération (17), qui est prévue dans la conduite d'aération de l'aspiration (14) et qui peut être commutée en fonction de la pression de fluide dans la conduite d'entrée d'aspiration (7),
**caractérisé en ce que** le deuxième réservoir de fluide (15; 15') peut être rempli par une conduite de remplissage (18), qui comporte une soupape de remplissage (19), dans lequel la conduite de remplissage (18) est raccordée par une extrémité (30) à la conduite d'irrigation (3).

2. Système (1) selon la revendication 1, dans lequel une autre extrémité (31) de la conduite de remplissage (18) se termine à une distance prédéterminée (20) d'un niveau de fluide maximum du deuxième réservoir de fluide (15), de telle manière qu'il n'existe aucun contact direct entre l'autre extrémité (31) de la conduite de remplissage (18) et le fluide (22) dans le deuxième réservoir de fluide (15).

3. Système (1) selon l'une quelconque des revendications précédentes, dans lequel il est prévu un capteur (16) pour détecter un niveau de fluide dans le deuxième réservoir de fluide (15).

4. Système selon l'une quelconque des revendications précédentes, dans lequel le deuxième réservoir de fluide (15) est relié à une conduite de trop-plein (23), par laquelle le fluide en excès peut être évacué dans un réservoir (24).

5. Système (1) selon la revendication 1, dans lequel la conduite de remplissage (18) est raccordée par une autre extrémité (31) au deuxième réservoir de fluide (15').

6. Système (1) selon la revendication 5, dans lequel un deuxième raccordement entre l'autre extrémité (31) de la conduite de remplissage (18) et le deuxième réservoir de fluide (15') peut être réalisé par des moyens de raccordement mécaniques ou chimiques ou par un procédé de raccordement thermique.

7. Système (1) selon l'une quelconque des revendications 5 ou 6, dans lequel le deuxième réservoir de fluide (15') présente une paroi avec au moins une zone élastiquement déformable.

8. Système (1) selon l'une quelconque des revendications 5 à 7, dans lequel le deuxième réservoir de fluide (15') est de forme tubulaire.

9. Système (1) selon l'une quelconque des revendications 5 à 8, dans lequel la conduite de remplissage (18), le deuxième réservoir de fluide (15') et la conduite d'aération de l'aspiration (14) sont réalisés d'une seule pièce.

10. Système (1) selon l'une quelconque des revendications précédentes, dans lequel la conduite d'irrigation (3) comporte une soupape d'irrigation (40).

11. Système (1) selon la revendication 3, dans lequel la soupape d'irrigation (40) est disposée dans la conduite d'irrigation (3), entre l'instrument (4) et la conduite de remplissage (18).

12. Système (1) selon l'une quelconque des revendications précédentes, dans lequel du fluide (22) peut être évacué par la conduite d'aération de l'aspiration (14) au fond ou à proximité du fond du deuxième réservoir de fluide (15; 15').

13. Système (1) selon l'une quelconque des revendications précédentes, dans lequel le capteur de pression (11) détecte la pression de fluide dans la conduite d'aspiration (6, 7), à proximité de l'instrument (4).

14. Système (1) selon l'une quelconque des revendications précédentes, dans lequel le capteur de pression (11) détecte la pression de fluide dans la conduite d'aspiration (6, 7), à l'intérieur de l'instrument (4), de préférence à proximité immédiate de la pointe (5) de l'instrument.
